# EUROPEAN PATENT APPLICATION

(11) **EP 4 613 277 A1**
(43) Date of publication of application: **10.09.2025**
(21) Application number: 23886086.0
(22) Date of filing: 19.10.2023
(51) Int. Cl.: A61K 35/747, A61P 25/28, A23L 33/135, C12N 1/20, C12R 1/225

(54) **COMPOSITION FOR PREVENTING, AMELIORATING, OR TREATING COGNITIVE DYSFUNCTION AND ALZHEIMER'S DISEASE COMPRISING LACTOBACILLUS FERMENTUM SRK414 STRAIN**

(30) Priority: 02.11.2022 KR 20220144820
(71) Applicant: CKD Bio Corporation, Seoul 03742 (KR)
(72) Inventor: CHA, Jiah, Anyang-si Gyeonggi-do 14043 (KR); LEE, In Ock, Siheung-si Gyeonggi-do 14994 (KR); KIM, Kyung Hwan, Seoul 01703 (KR); KIM, Byoung-Kook, Seoul 08048 (KR); SHIN, Chang-Hun, Hwaseong-si Gyeonggi-do 18237 (KR); MOOK, Inhee, Seoul 04386 (KR); CHOI, Hyunjung, Seoul 02725 (KR)
(74) Representative: J A Kemp LLP
(86) International application number: PCT/KR2023/016249
(87) International publication number: WO 2024/096389

(57) **Abstract**

The present invention relates to a composition for preventing, ameliorating or treating cognitive dysfunction or Alzheimer's disease comprising, as an active ingredient, Lactobacillus fermentum SRK414 strain which has accession number KCTC13687BP. The strain of the present invention is excellent in reducing barrier permeability and reducing amyloid beta and tau proteins and has an excellent effect in improving cognitive function, and accordingly, the strain can be useful for a food and therapeutic agent for same purposes.

## Description

### Technical Field

The present disclosure claims priority to Korean Patent Application No. 10-2022-0144820, filed with the Korean Intellectual Property Office on November 2, 2022, the entire disclosure of which is incorporated herein by reference.

The present disclosure relates to a composition for preventing, alleviating, or treating cognitive dysfunction and Alzheimer's disease, comprising *Lactobacillus fermentum* SRK414 strain.

### Background Art

Alzheimer's disease (AD) is a degenerative brain disorder that constitutes the most common form of dementia. Patients with AD experience progressive deterioration of various cognitive functions, beginning with early-stage memory loss and advancing to impairments in language ability, judgment, and other cognitive faculties, ultimately leading to significant disruptions in daily life. Although the precise etiology and pathogenesis of Alzheimer's disease have not yet been fully elucidated, the condition is known to be primarily associated with neuronal damage caused by the abnormal accumulation of amyloid beta (Aβ) protein and tau protein in brain tissue, as well as excessive activation of immune cells in the brain triggered by such damage. As a representative neurodegenerative disease explained by these major hypotheses, most currently available therapeutics for AD are aimed at addressing impaired neurotransmission between neurons. Consequently, research and development of therapeutic agents have largely focused on targets within the brain.

According to numerous studies, gut microbiota play a critical role in bidirectional communication between the gut and the brain through the endocrine, nervous, and immune systems. Changes in the composition of gut microbiota and their metabolic byproducts have been shown to directly affect not only brain functions such as memory and learning but also systemic processes such as appetite, sleep, and mood regulation, via immune responses, hormonal signaling, and neurotransmitters. As the connection between the gut and the brain has become increasingly evident, a growing number of studies have reported the involvement of gut microbiota in various neurological disorders. This bidirectional communication system is often referred to as the "gut-brain axis." Based on this theory, active research is being conducted to identify candidate microorganisms for the treatment of neurodegenerative diseases, such as Parkinson's disease and Alzheimer's disease, using gut microbiota.

### Disclosure of Invention

### Technical Problem

The inventors of the present disclosure have made extensive research efforts to develop a substance capable of preventing, alleviating, or treating cognitive dysfunction and Alzheimer's disease using gut microbiota. As a result, they have identified that Lactobacillus fermentum SRK414 strain improves intestinal barrier permeability, reduces levels of amyloid beta and tau proteins-which are biomarkers of neurodegenerative diseases such as Alzheimer's disease-and enhances cognitive function in animal models. Through these findings, the present disclosure has been completed.

Accordingly, an object of the present disclosure is to provide a composition for preventing, alleviating, or treating cognitive dysfunction or Alzheimer's disease, comprising *Lactobacillus fermentum* SRK414 strain, which has been deposited under accession number KCTC13687BP, as an active ingredient.

Another object of the present disclosure is to provide the use of *Lactobacillus fermentum* SRK414 strain, deposited under accession number KCTC13687BP, for preventing, alleviating, or treating cognitive dysfunction or Alzheimer's disease.

### Solution to Problem

According to one aspect of the present disclosure, there is provided a pharmaceutical composition for preventing or treating cognitive dysfunction or Alzheimer's disease, comprising *Lactobacillus fermentum* SRK414 strain, which has been deposited under accession number KCTC13687BP, as an active ingredient.

According to another aspect of the present disclosure, there is provided a food composition for preventing or alleviating cognitive dysfunction or Alzheimer's disease, comprising *Lactobacillus fermentum* SRK414 strain, which has been deposited under accession number KCTC13687BP, as an active ingredient.

The inventors of the present disclosure have newly isolated *Lactobacillus fermentum* SRK414 strain (accession number: KCTC13687BP), and have previously confirmed that fermented milk prepared using this strain increases bone density and bone ratio in an experimental animal model of secondary osteoporosis. [Refer to Korean Registered Patent Publication No. 10-2120479 (published on June 9, 2020)].

In the present disclosure, *Lactobacillus fermentum* SRK414 strain has been deposited with the Korean Collection for Type Cultures (KCTC) of the Korea Research Institute of Bioscience and Biotechnology under accession number KCTC13687BP.

The characteristics of the *Lactobacillus fermentum* SRK414 strain are as follows:
- It comprises the 16S rRNA sequence of Sequence ID No: 1.
- It is a Gram-positive rod-shaped or coccus-shaped bacillus.
- It is a part of the normal microbiota in the human oral cavity, gastrointestinal tract, and female reproductive organs, and is generally regarded as safe for use in foods, including probiotics and fermented foods. *Lactobacillus fermentum* belongs to the genus *Lactobacillus,* members of which are known for their diverse applications. These applications include fermentation of food and feed. Some strains of *L. fermentum* have been reported to exhibit intrinsic resistance to certain antibiotics and chemotherapeutic agents.

In one embodiment of the present disclosure, the cognitive dysfunction may be accompanied by symptoms such as memory impairment, reduced attention, impaired spatial perception, impaired language ability, or a combination thereof.

In another embodiment of the present disclosure, the cognitive dysfunction may be an amyloid-beta accumulation disorder selected from the group consisting of Alzheimer's disease, Parkinson's disease dementia, Lewy body dementia, Huntington's disease dementia, preclinical Alzheimer's disease, and Down syndrome, but it is not limited thereto.

In another embodiment of the present disclosure, the cognitive dysfunction may be a tauopathy selected from the group consisting of corticobasal degeneration (CBD), progressive supranuclear palsy (PSP), Pick's disease, and frontotemporal dementia (FTD), but it is not limited thereto.

In one embodiment of the present disclosure, the *Lactobacillus fermentum* SRK414 strain inhibits the production of amyloid beta protein, tau protein, or a combination thereof in brain tissue.

In one embodiment of the present disclosure, the amyloid beta protein may be soluble or insoluble amyloid beta protein.

In one embodiment of the present disclosure, the amyloid beta protein may be Aβ40, Aβ42, or a combination thereof, but it is not limited thereto.

In one embodiment of the present disclosure, the tau protein may be phosphorylated tau protein.

In one embodiment of the present disclosure, the tau protein may include, but is not limited to, Tau13, phospho-Tau (Thr231), phospho-Tau (Ser202, Thr205), phospho-Tau (Thr181), phospho-Tau (Thr212, Ser214), phospho-Tau (Ser396), and phospho-Tau (Ser422).

In one embodiment of the present disclosure, the tau protein may be soluble or insoluble tau protein.

In one embodiment of the present disclosure, the composition exhibits an effect of reducing intestinal permeability compared to a control group not treated with the strain.

In one embodiment of the present disclosure, the composition results in an increase in the abundance of strains belonging to *Eubacterium xylanophilum, Ruminococcaceae, Alloprevotella,* or a combination thereof in the gut microbiota, compared to a control group not treated with the strain.

In one embodiment of the present disclosure, the composition results in a decrease in the abundance of strains belonging to *Peptococcaceae, Lachnospiraceae* NK4A136 group, *Clostridia, Staphylococcus,* or a combination thereof in the gut microbiota, compared to a control group not treated with the strain.

The active ingredient of the composition of the present disclosure, i.e., the above-mentioned strain, may be in the form of an isolated and/or purified bacterial body, or in the form of a culture containing the bacterial body, a lysate thereof, an extract thereof, a culture supernatant, or a concentrate, concentrate product, dried product, or, if necessary, a diluted solution or diluted product. It includes all forms obtained by processing the above-mentioned culture or cultured product.

The methods of culturing, extracting, isolating, concentrating, drying, and diluting the bacterial body are not particularly limited.

The culture medium for culturing the bacterial strain may typically include milk proteins such as skim milk, whey, and casein, saccharides, and yeast extract. Various conventional aerobic or anaerobic culturing methods may be appropriately employed.

The culturing temperature may be set, for example, at 35°C to 45°C. During the culturing process, a neutralization culturing method may be used in which the pH of the medium is maintained in an acidic range, for example, at a pH of about 5 to 6, by adding an alkali such as sodium hydroxide to adjust the pH from neutral. In addition to the neutralization culturing method, other culturing methods such as batch culturing may be employed as appropriate. After culturing, the culture or the supernatant may be concentrated, dried, or diluted, if necessary.

Furthermore, the supernatant and bacterial cells may be separated by methods such as centrifugation or membrane separation, and the bacterial cells may be recovered in a concentrated form. The components within the bacterial cells may be extracted by ultrasonic treatment or enzymatic treatment. The culture, supernatant, bacterial cells, or extracts thereof may also be dried. These may be used as the active ingredient in the composition of the present disclosure.

When the composition of the present disclosure is prepared as a food composition, in addition to the lactic acid bacterium as the active ingredient, it may further include ingredients that are conventionally added during food manufacturing. Such ingredients may include, for example, proteins, carbohydrates, fats, nutrients, seasonings, and flavoring agents. The carbohydrates may include monosaccharides (e.g., glucose, fructose), disaccharides (e.g., maltose, sucrose, oligosaccharides), and polysaccharides (e.g., dextrin, cyclodextrin), as well as sugar alcohols such as xylitol, sorbitol, and erythritol. As flavoring agents, natural flavoring substances (e.g., thaumatin, stevia extracts such as rebaudioside A, glycyrrhizin) and synthetic sweeteners (e.g., saccharin, aspartame) may be used.

For example, when the food composition of the present disclosure is formulated as a drink, it may further include, in addition to the strain as the active ingredient, citric acid, high fructose corn syrup, sugar, glucose, acetic acid, malic acid, fruit juice, jujube extract, or licorice extract.

The food composition of the present disclosure encompasses all processed forms of natural materials, including food, functional food, nutritional supplements, health food, and food additives. These types of food compositions may be manufactured in various forms according to methods known in the art.

For instance, the lactic acid bacterium may be formulated as a health food in the form of a tea, juice, or drink for oral intake, or it may be processed into granules, capsules, or powders. In terms of food products, it may be added to beverages (including alcoholic beverages), fruits and processed fruit products (e.g., canned fruits, preserved fruits, jam, marmalade), fish, meat and processed meat products (e.g., ham, sausage, corned beef), baked goods and noodles (e.g., udon, buckwheat noodles, ramen, spaghetti, macaroni), fruit juice, various types of drinks, cookies, taffy, dairy products (e.g., yogurt, fermented milk, butter, cheese), edible vegetable oils, margarine, vegetable protein products, retort foods, frozen foods, and various seasonings (e.g., soybean paste, soy sauce, sauces). In addition, the lactic acid bacterium of the present disclosure may be used in the form of a food additive, and for such purposes, it may be manufactured in the form of a powder or concentrate.

When the composition of the present disclosure is formulated as a pharmaceutical composition, the pharmaceutical composition may comprise a pharmaceutically acceptable carrier. The pharmaceutically acceptable carrier included in the pharmaceutical composition of the present disclosure refers to one that is commonly used in the preparation of pharmaceutical formulations, and may include, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylparaben, propylparaben, talc, magnesium stearate, and mineral oil. In addition to the above components, the pharmaceutical composition of the present disclosure may further comprise lubricants, wetting agents, sweeteners, flavoring agents, emulsifiers, suspending agents, preservatives, and the like.

The pharmaceutical composition of the present disclosure may be administered orally or non-orally, and is preferably administered orally. The pharmaceutical composition of the present disclosure may be formulated into various oral or non-oral dosage forms, including but not limited to the following.

Oral dosage forms may include, for example, tablets, pills, hard or soft capsules, liquids, suspensions, emulsions, syrups, granules, and elixirs. These dosage forms may contain, in addition to the active ingredient, one or more diluents or excipients commonly used in the art, such as fillers, extenders, wetting agents, disintegrants, lubricants, binders, and surfactants. Disintegrants may include agar, starch, alginic acid or sodium salts thereof, and dicalcium phosphate anhydrous. Lubricants may include silica, talc, stearic acid or magnesium or calcium salts thereof, and polyethylene glycol. Binders may include magnesium aluminum silicate, starch paste, gelatin, tragacanth, methylcellulose, sodium carboxymethylcellulose, polyvinylpyrrolidone, and low-substituted hydroxypropylcellulose. Other diluents may include lactose, dextrose, sucrose, mannitol, sorbitol, cellulose, and glycine. If necessary, other commonly known additives such as boiling mixtures, absorbents, coloring agents, flavoring agents, and sweeteners may also be used.

The composition described herein may be sterilized or may include preservatives, stabilizers, hydrating agents, emulsifying aids, salts for osmotic regulation, buffers, and other therapeutically useful substances. It may be formulated using conventional techniques such as mixing, granulation, or coating.

The appropriate dosage of the pharmaceutical composition of the present invention may vary depending on several factors, including the formulation method, route of administration, age, weight, sex, pathological condition of the patient, food intake, time of administration, administration route, rate of excretion, and responsiveness to the treatment.

The pharmaceutical composition of the present invention may be formulated into a unit dosage form or filled into a multi-dose container using pharmaceutically acceptable carriers and/or excipients, in accordance with methods readily practiced by those skilled in the art. The dosage form may be in the form of a solution, suspension, syrup, or emulsion in an oil or aqueous medium, or in the form of an extract, powder, granule, tablet, or capsule, and may additionally contain dispersing or stabilizing agents.

According to another embodiment of the present invention, the present invention provides a method for preventing, alleviating, or treating cognitive impairment or Alzheimer's disease, comprising administering to a subject *Lactobacillus fermentum* SRK414 strain deposited under Accession No. KCTC13687BP.

As used herein, the term "subject" refers to an individual in need of prevention, alleviation, or treatment of cognitive impairment or Alzheimer's disease, but is not limited thereto. Specifically, the subject may exhibit symptoms such as memory decline, decreased attention, impaired spatial perception, reduced language ability, or any combination thereof, or may be an individual diagnosed with Alzheimer's disease.

According to another embodiment of the present invention, the present invention provides a method for preventing or treating cognitive impairment or Alzheimer's disease, comprising administering to a subject a pharmaceutical composition comprising *Lactobacillus fermentum* SRK414 strain, deposited under Accession No. KCTC13687BP, as an active ingredient.

According to yet another embodiment of the present invention, the present invention provides a method for preventing or improving cognitive impairment or Alzheimer's disease, comprising administering to a subject a food composition comprising *Lactobacillus fermentum* SRK414 strain, deposited under Accession No. KCTC13687BP, as an active ingredient.

### Effect of the Invention

The present invention provides a composition for the prevention, alleviation, or treatment of cognitive impairment and Alzheimer's disease, comprising Lactobacillus fermentum SRK414 strain. The strain of the present invention exhibits excellent efficacy in reducing intestinal barrier permeability, as well as in decreasing levels of amyloid-beta and tau proteins. Furthermore, it demonstrates a significant effect in improving cognitive function, thereby rendering it useful as a food or therapeutic agent for such purposes.

### Brief Description of Drawings

FIG. 1 and FIG. 2 show the results of microbiota analysis in the gut of Alzheimer's disease animal models after administration of the SRK414 strain of the present invention.
FIG. 3 illustrates the intestinal barrier permeability in the experimental animals following administration of the SRK414 strain in Alzheimer's disease animal models.
FIG. 4 shows the results of amyloid-beta immunohistochemical staining in brain tissue after administration of the SRK414 strain in Alzheimer's disease animal models.
FIG. 5 presents the ELISA results for amyloid-beta levels in brain tissue following administration of the SRK414 strain in Alzheimer's disease animal models.
FIG. 6 and FIG. 7 illustrate the Western blot results for tau protein expression in brain tissue after administration of the SRK414 strain in Alzheimer's disease animal models.
FIG. 8 depicts the improvement in short-term memory and spatial perception abilities in experimental animals, assessed via the Y-maze test, after administration of the SRK414 strain in Alzheimer's disease animal models.

### Mode for Carrying out the Invention

Hereinafter, the disclosure will be described in more detail through exemplary embodiments. These exemplary embodiments are provided only for the purpose of specifically illustrating the disclosure, and therefore, according to the purpose of the disclosure, it would be apparent to a person skilled in the art that these exemplary embodiments are not construed to limit the scope of the disclosure.

### Examples

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Example 1. Preparation of Probiotics

The *Lactobacillus fermentum* SRK414 strain used in this experiment was isolated from the feces of an infant. The lactic acid bacteria seed culture was cultivated in a flask containing MRS broth at 37°C for 24 hours. The seed culture was then inoculated into a self-formulated optimized medium. The culture was maintained at a pH of 5.5 to 6.0 and stirred at 55 to 65 RPM for 18 to 20 hours at 37°C. Freeze-drying of the 40× concentrated cells was performed in accordance with an in-house protocol. After freeze-drying, the number of colony-forming units (CFUs) per 1 g of probiotic powder was determined by serial dilution. The probiotics were suspended in 1× PBS and adjusted to a concentration of 5×10⁹ CFU/200 µL prior to use.

### Example 2. Generation of Alzheimer's Disease Animal Model and Experimental Group Setup

An Alzheimer's disease-like pathology (ADLP) transgenic mouse model was generated as follows: i) 5XFAD (Tg6977, Jackson Laboratory, Stock#006554) mice expressing the human APP gene carrying Swedish (K670N/M671L), Florida (I716V), and London (V717I) mutations under control of the Thy1 promoter, and the human PSEN1 gene carrying M146L and L286V mutations; and ii) JNPL3 (TauP301L-JNPL3, Taconic, Stock#2508 homozygote) mice expressing the human Tau gene with a P301L mutation under control of the prion protein promoter, were crossed.

The resulting ADLP^{APT} mice, expressing three different mutant human genes, were used in the experiments. Since female ADLP^{APT} mice develop Alzheimer's-related pathological changes and cognitive impairments earlier than males, only female mice were used in this study.

To evaluate the therapeutic effect of the SRK414 strain, 2-2.5-month-old ADLPAPT mice were administered 200 µL of either SRK414 suspended in PBS (SRK414 group) or PBS alone (negative control group, NC). Additionally, wild-type ADLPWT mice received 200 µL of PBS (WT group). Oral administration was performed five times per week for a duration of five months.

### Example 3. Evaluation of Gut Microbiota Composition

To evaluate the gut microbiota of the experimental animals, fecal samples were collected prior to sacrifice and stored at -80°C until analysis. Genomic DNA was extracted from the fecal samples using the QIAamp DNA Stool Mini Kit (Qiagen, 51304). The V3-V4 region of the 16S rRNA gene was amplified by PCR using the 341F primer (5'-CCTACGGGNGGCWGCAG-3') and the 805R primer (5'-GACTACHVGGGTATCTAATCC-3'). The amplified PCR products were purified using HiAccuBead (AccuGene, ACN01.50). Metagenomic sequencing was performed using the Ion Torrent S5 Sequencer System.

The resulting sequence data were analyzed using the QIIME2 pipeline (version 2022.2) to assess the overall gut microbial profile. To identify taxonomic differences between the SRK414-administered group and the negative control (NC) group, the Linear Discriminant Analysis Effect Size (LEfSe) algorithm was used at the genus level.

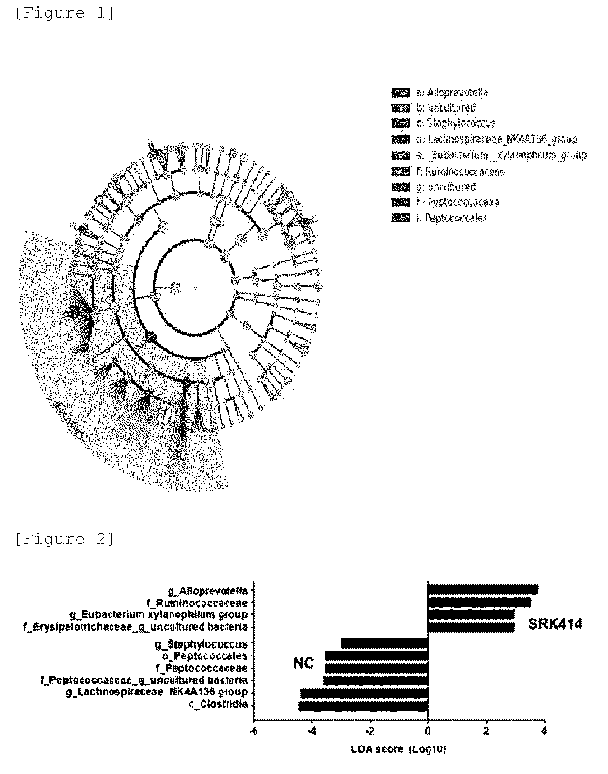

The results are presented in FIGS. 1 and 2.

As shown in FIGS. 1 and 2, the SRK414-treated group exhibited upregulation of *Eubacterium xylanophilum group, Ruminococcaceae,* and *Alloprevotella,* while *Peptococcaceae, Lachnospiraceae NK4A136 group, Clostridia,* and *Staphylococcus* were downregulated.

### Example 4. Evaluation of Intestinal Barrier Permeability

To assess the permeability of the intestinal barrier in experimental animals, a 4 kDa fluorescent tracer was used. Prior to the evaluation, mice were fasted for 4 hours, followed by oral administration of the fluorescent compound at a dose of 0.6 mg/g of body weight. Two hours after administration, the intestinal contents were visualized and the residual fluorescent signal was measured to evaluate intestinal barrier permeability.

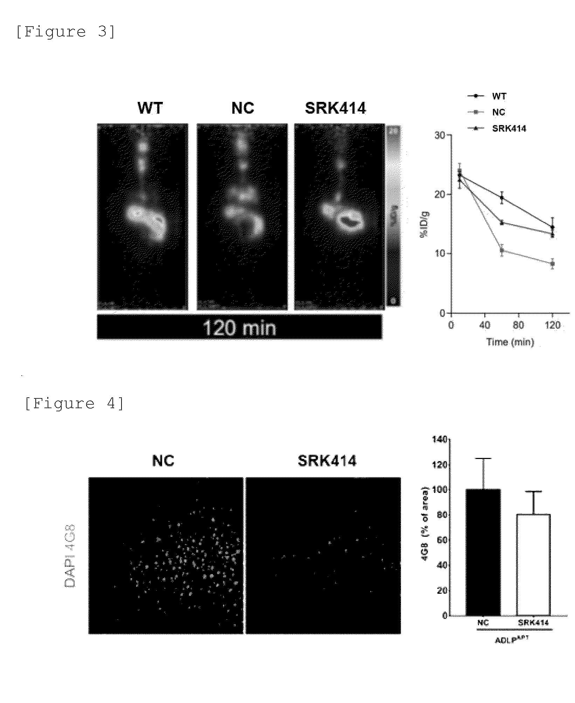

The results are presented in FIG. 3.

As shown in FIG. 3, ADLPAPT mice exhibited increased intestinal permeability compared to wild-type mice, evidenced by a significant decrease in the residual fluorescence in the intestinal tract 2 hours after administration. In contrast, the group treated with the SRK414 strain showed increased residual fluorescence compared to the NC group, indicating that the SRK414 strain effectively reduced intestinal permeability.

### Example 5. Immunohistochemistry and ELISA for Aβ Analysis

### 5-1. Immunohistochemistry (IHC)

Experimental animals were anesthetized with a mixture of Tiletamine-Zolazepam and Xylazine (1.2 mg/kg) and perfused with PBS. Brain tissues were fixed in 4% paraformaldehyde for 24 hours and dehydrated in a 30% sucrose solution for approximately 72 hours. The pretreated brain tissues were frozen at -80°C, and coronal sections were obtained using a cryostat maintained at - 25°C. The brain sections were washed with PBS, followed by blocking and permeabilization. They were then incubated with the following primary antibodies: Biotin-labeled 4G8 (1:700, Covance, SIG-39240), GFAP (1:1,000, Invitrogen, 13-0300), and Iba1 (1:500, Wako, 019-19741). After washing with PBS, the sections were further incubated with fluorescent dye-conjugated secondary antibodies and observed using a confocal microscope.

The results are shown in FIG. 4.

As illustrated in FIG. 4, the area occupied by Aβ plaques in the brains of animals administered with strain SRK414 was significantly reduced compared to the NC group. This demonstrates that strain SRK414 is effective in ameliorating Aβ pathology in the brain.

### 5-2. Human Aβ ELISA Assay

Experimental animals were anesthetized as described above and perfused with PBS. The brain tissues were homogenized in RIPA buffer (50 mM Tris-HCl, pH 7.4; 150 mM NaCl; 1% Nonidet P-40; 0.1% SDS; 0.5% sodium deoxycholate) for the ELISA assay. Protein concentration in the supernatant was quantified, and 100 µg of protein was ultracentrifuged to separate RIPA-soluble and RIPA-insoluble fractions. The RIPA-insoluble fraction was resuspended in 70% formic acid and neutralized with 1 M Tris-base before ELISA. The concentrations of RIPA-soluble Aβ40 and Aβ42, as well as RIPA-insoluble Aβ40 and Aβ42, were determined using a human Aβ-specific ELISA kit in accordance with the manufacturer's instructions.

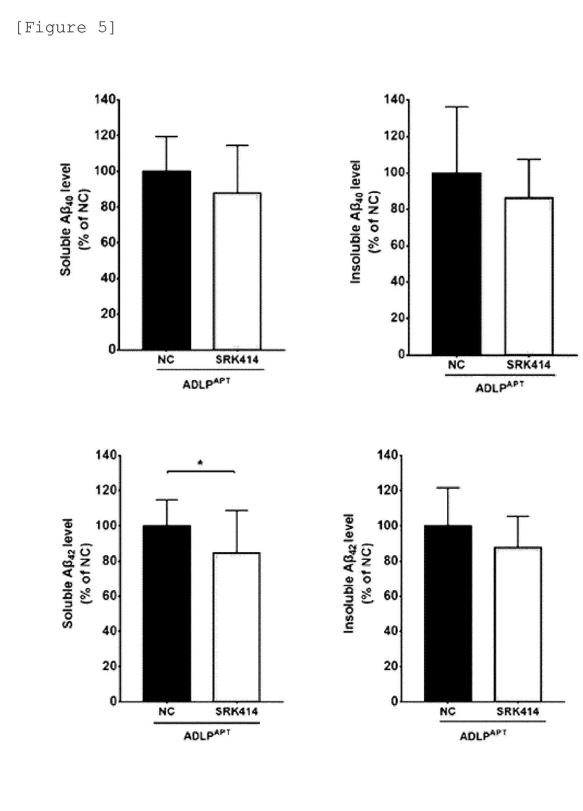

The results are shown in FIG. 5.

As shown in FIG. 5, a significant reduction in soluble Aβ42 levels was observed in the SRK414-treated group compared to the NC group. In addition, levels of soluble Aβ40, insoluble Aβ40, and Aβ42 showed a decreasing trend, suggesting that strain SRK414 contributes to the reduction of Aβ pathology in the brain.

### Example 6. Tau Protein Analysis via Western Blotting

### 6-1. Sarkosyl-Insoluble Fractionation

Experimental animals were anesthetized with a mixture of Tiletamine-Zolazepam and Xylazine (1.2 mg/kg) and perfused with PBS. Brain tissues were homogenized in Tris-buffered saline (TBS; 150 mM NaCl; 25 mM Tris-HCl, pH 7.4; 1 mM EDTA; 1 mM EGTA). The supernatant of the tissue lysate was mixed with 1% N-Lauroylsarcosine sodium salt solution and incubated at 37°C for 1 hour. After incubation, the samples were ultracentrifuged to separate into sarkosyl-soluble and sarkosyl-insoluble fractions.

### 6-2. Western Blotting

The sarkosyl-soluble fraction was quantified for protein content and used for western blot analysis. The sarkosyl-insoluble fraction was resuspended in 5X sample buffer and boiled at 70°C for 10 minutes prior to western blotting. Proteins in the samples were separated by sodium dodecyl sulfate-polyacrylamide gel electrophoresis (SDS-PAGE) and transferred to a polyvinylidene fluoride (PVDF) membrane. For immunoblotting, membranes were blocked with 5% skim milk and incubated overnight at 4°C with the following primary antibodies: APP (6E10, 1:1,000, Covance, SIG-39320), Tau13 (1:2,000, Abcam, ab13090), phospho-Tau (Thr231; AT180, 1:1,000, Invitrogen, MN1040), phospho-Tau (Ser202, Thr205; AT8, 1:1,000, Invitrogen, MN1020), phospho-Tau (Thr181; AT270, 1:1,000, Invitrogen, MN1050), phospho-Tau (Thr212, Ser214; AT100, 1:1,000, Invitrogen, MN1060), phospho-Tau (Ser396, 1:2,000, Thermo Fisher Scientific, 44-752G), phospho-Tau (Ser422, 1:1,000, Thermo Fisher Scientific, 44-764ZG), and β-actin (1:2,000, Cell Signaling Technology, #3700). After incubation, membranes were washed with Tris-buffered saline containing 0.05% Tween 20 (TBST) and incubated with horseradish peroxidase (HRP)-conjugated secondary antibodies at room temperature for 1 hour. Chemiluminescence signals were developed using an enhanced chemiluminescent substrate (ECL; Abfrontier, LF-QC0103), and detected using an image analyzer (LAS-3000; Fujifilm Corporation). Band intensities were quantified using Multigauge software (Fujifilm Corporation).

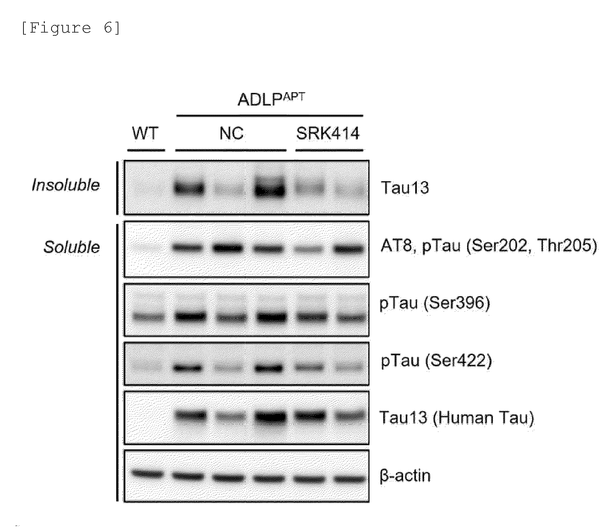

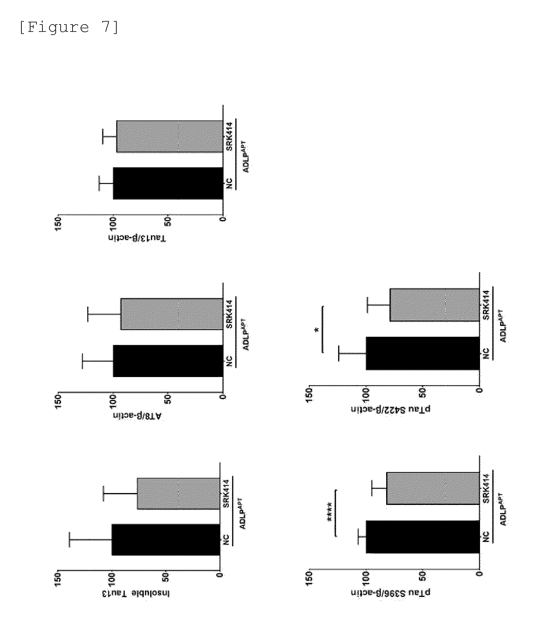

The results are shown in **FIGS. 6** **and** **7****.**

As illustrated in FIGS. 6 and 7, phosphorylated Tau at Ser396 was significantly reduced in the SRK414-treated group compared to the NC group. In addition, there was a decreasing trend in insoluble Tau13, phosphorylated Tau at Ser422, and Ser202/Thr205, indicating the potential of SRK414 in alleviating Tau pathology.

### Example 7. Y-Maze Test

To evaluate the short-term memory and spatial recognition abilities of the experimental animals, a Y-maze test was performed. One day prior to the test, the animals were acclimated to the testing room and apparatus. On the test day, each animal was placed at the center of the Y-maze and allowed to freely explore all three arms of the maze for 8 minutes. The total number of entries was defined as the number of times the animal entered any arm of the maze. Spontaneous alternation was defined as consecutive entries into three different arms. The percentage of alternation was calculated as follows: Alternation (%) = [Number of Alternations / (Total Entries - 2)] × 100

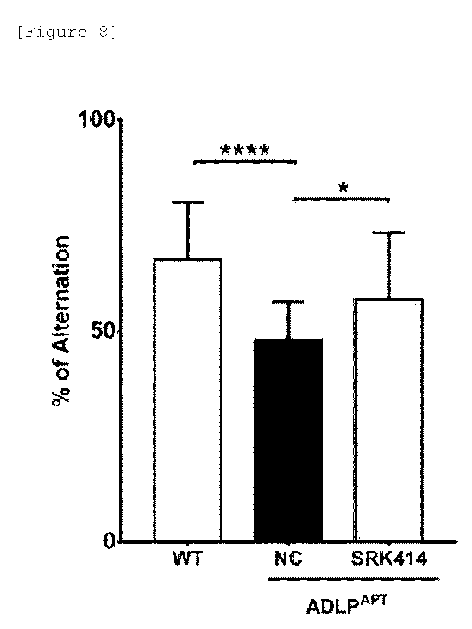

The results are shown in FIG. 8.

As illustrated in FIG. 8, the group treated with the SRK414 strain exhibited a significantly higher alternation percentage compared to the NC group, indicating that administration of the SRK414 strain improved short-term memory and spatial recognition ability in the animal model.

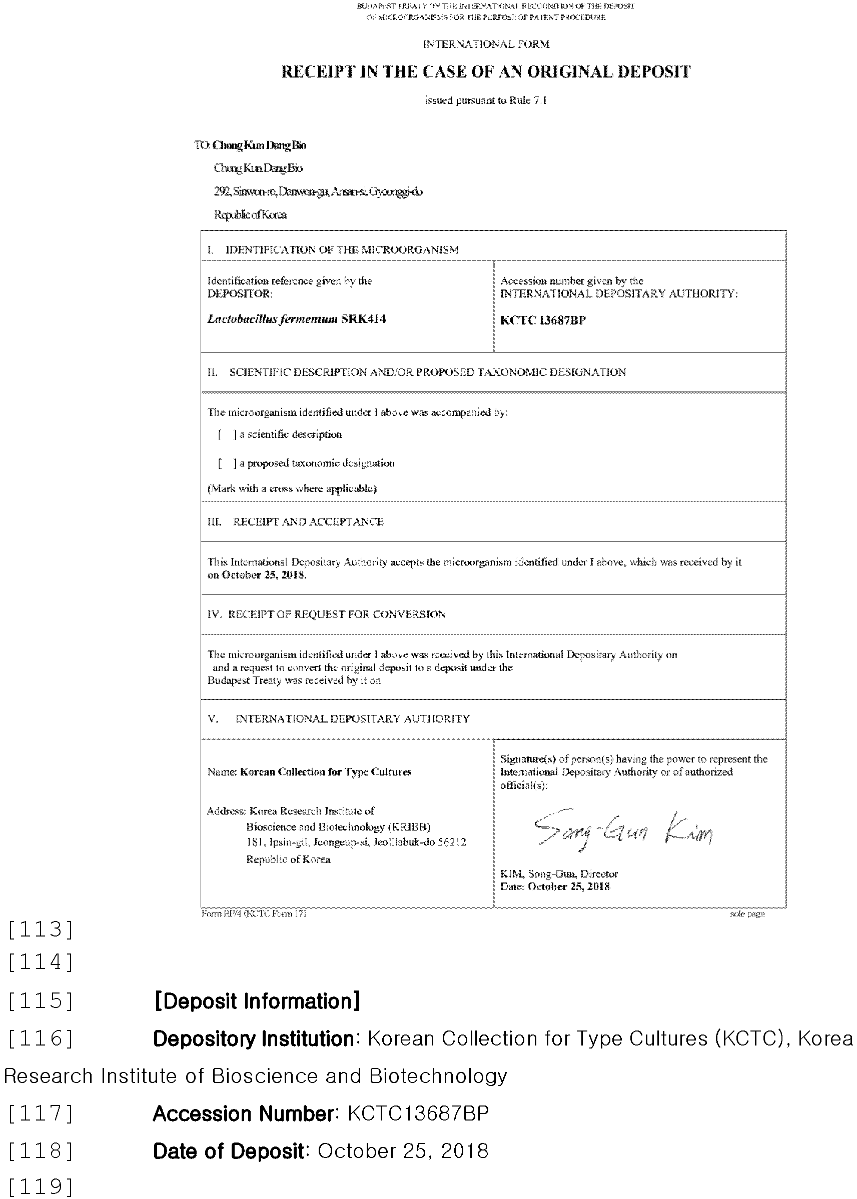
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Chong Kun Dang Bio
Chong Kun Dang Bio
292, Sinwon-ro, Danwon-gu, Ansan-si, Gyeonggi-do
Republic of Korea

I. IDENTIFICATION OF THE MICROORGANISM
Identification reference given by the
DEPOSITOR:

Lactobacillus fermentum SRK414 | Accession number given by the
INTERNATIONAL DEPOSITARY AUTHORITY:

KCTC 13687BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on October 25, 2018.
IV. RECEIPT OF REQUEST FOR CONVERSION
The microorganism identified under I above was received by this International Depositary Authority on
and a request to convert the original deposit to a deposit under the
Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):


KIM, Song-Gun, Director
Date: October 25, 2018
Form BP/4 (KCTC Form 17) sole page

[113]
[114]
[115] [Deposit Information]
[116] Depository Institution: Korean Collection for Type Cultures (KCTC), Korea
Research Institute of Bioscience and Biotechnology
[117] Accession Number: KCTC13687BP
[118] Date of Deposit: October 25, 2018
[119]

## Claims

1. A pharmaceutical composition for the prevention or treatment of cognitive dysfunction or Alzheimer's disease, comprising *Lactobacillus fermentum* SRK414 strain deposited under accession number KCTC13687BP as an active ingredient.

2. The pharmaceutical composition according to claim 1, wherein the cognitive dysfunction is accompanied by symptoms selected from the group consisting of memory loss, decreased attention, impaired spatial perception, impaired language ability, or a combination thereof.

3. The pharmaceutical composition according to claim 1, wherein the *Lactobacillus fermentum* SRK414 strain inhibits the production of amyloid beta protein, tau protein, or a combination thereof in brain tissue.

4. The pharmaceutical composition according to claim 1, wherein the amyloid beta protein is a soluble or insoluble amyloid beta protein.

5. The pharmaceutical composition according to claim 1, wherein the tau protein is a phosphorylated tau protein.

6. The pharmaceutical composition according to claim 1, wherein the composition reduces intestinal permeability compared to a control group not treated with the strain.

7. The pharmaceutical composition according to claim 1, wherein the composition increases the abundance of gut microbiota belonging to *Eubacterium xylanophilum, Ruminococcaceae, Alloprevotella,* or a combination thereof, compared to a control group not treated with the strain.

8. The pharmaceutical composition according to claim 1, wherein the composition reduces the abundance of gut microbiota belonging to *Peptococcaceae, Lachnospiraceae NK4A136 group, Clostridia, Staphylococcus,* or a combination thereof, compared to a control group not treated with the strain.

9. A food composition for the prevention or improvement of cognitive dysfunction or Alzheimer's disease, comprising *Lactobacillus fermentum* SRK414 strain deposited under accession number KCTC13687BP as an active ingredient.
